# EUROPEAN PATENT SPECIFICATION

(11) **EP 4 643 936 B1**
(45) Date of publication and mention of the grant of the patent: **09.09.2026**
(21) Application number: 25173906.6
(22) Date of filing: 02.05.2025
(51) Int. Cl.: A61N 1/372, A61N 1/375, A61N 1/05

(54) **BIOSTIMULATOR TRANSPORT SYSTEM HAVING RELEASE MECHANISM**
BIOSTIMULATORTRANSPORTSYSTEM MIT FREIGABEMECHANISMUS
SYSTÈME DE TRANSPORT DE BIOSTIMULATEUR AYANT UN MÉCANISME DE LIBÉRATION

(30) Priority: 03.05.2024 US 202463642596 P; 05.11.2024 US 202463716649 P; 30.04.2025 US 202519195611
(43) Date of publication of application: 05.11.2025
(73) Proprietor: Pacesetter, Inc., Sylmar, CA 91342 (US)
(72) Inventor: ARNAR, Bernhard, Sylmar, CA 91342 (US); JACKSON, Aaron, Sylmar, CA 91342 (US); SACHA, Mike, Sylmar, CA 91342 (US); TERWEY, Russ, Sylmar, CA 91342 (US)
(74) Representative: Barker Brettell Sweden AB

(56) References cited:
- EP-B1- 3 954 432
- US-A1- 2020 253 614
- US-A1- 2022 023 646
- US-A1- 2022 117 735
- US-A1- 2023 241 348
- US-A1- 2024 001 112
- US-B2- 10 842 530

## Description

### TECHNICAL FIELD

The present disclosure relates to biostimulators and related biostimulator systems. More specifically, the present disclosure relates to biostimulator transport systems having release mechanisms to attach to and release from biostimulators.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

US 2022/023646 A1 discloses a leadless biostimulator including an attachment feature to facilitate precise manipulation during delivery or retrieval. The attachment feature can be monolithically formed from a rigid material, and includes a base, a button, and a stem interconnecting the base to the button. The stem is a single post having a transverse profile extending around a central axis. The transverse profile can be annular and can surround the central axis. The leadless biostimulator includes a battery assembly having a cell can that includes an end boss. A tether recess in the end boss is axially aligned with a face port in the button to receive tethers of a delivery or retrieval system through an inner lumen of the stem. The attachment feature can be mounted on and welded to the cell can at a thickened transition region around the end boss.

US 2024/001112 A1 discloses a biostimulator transport system, such as a biostimulator delivery system. The biostimulator transport system includes a flexible tether extending through a tether support to a tether bight. The tether bight loops through an attachment feature of a biostimulator to connect the biostimulator to the biostimulator transport system. Tether legs extend from the tether bight to a handle of the biostimulator transport system. The handle includes a housing and a knob, and the tether legs attach to the housing and the knob. A tether leg can be disconnected from the housing and the knob can be removed to pull the tether out of the attachment feature and release the biostimulator from the biostimulator transport system.

### SUMMARY

Existing biostimulator transport systems used for delivery or retrieval of leadless cardiac pacemakers may have a set of tethers that include end features to engage an attachment feature of a leadless biostimulator. The tethers can be moved relative to each other to align or misalign the end features. In the aligned state, the end features can lock the tethers to the leadless biostimulator. In the misaligned state, the end features can unlock the tethers from the leadless biostimulator. Accordingly, adjustment of the end features can retain or release the leadless biostimulator.

The tethers, end features, and accompanying mechanisms of existing biostimulator transport systems can be complex and expensive. More particularly, the mechanisms and components used to implement the biostimulator retention/release feature of existing biostimulator transport systems may require precise movements and fine mechanical tolerances. Furthermore, the cyclic loading seen by the biostimulator transport system within the target anatomy can challenge the precise movements and complicate delivery of the leadless biostimulator. Accordingly, biostimulator transport systems would benefit from mechanisms of retention and release that are simple, inexpensive, and reliable.

Existing biostimulator transport systems engage and transmit torque to the attachment feature of the leadless cardiac pacemaker to drive the biostimulator into a target tissue. Available space in the target anatomy can be limited and, thus, reducing an overall length of the torque transmission features of the transport system and the biostimulator can be advantageous. Furthermore, torque transmission by existing biostimulator transport systems is provided through structures that can bind the biostimulator transport system to the leadless cardiac pacemaker. When the structures jam, delivery and release of the biostimulator at the target tissue can be impeded. The jamming can be caused by misalignment of the structures, which can cause uneven distribution of torque around the attachment feature. Accordingly, biostimulator transport systems and biostimulators can benefit from torque transmission elements that have minimal length and evenly distribute torque around an attachment feature of the biostimulator.

A biostimulator transport system is described. In an embodiment, the biostimulator transport system includes a locking tube having a central lumen. The locking tube has a distal tube section having a conical profile. The biostimulator transport system includes a tether extending through the central lumen. The tether includes a locking end coupled to a tether body. The locking end is wider than the tether body.

A biostimulator is described. In an embodiment, the biostimulator includes a housing containing circuitry. The biostimulator includes a fixation element coupled to a distal end of the housing. The biostimulator includes an attachment feature coupled to a proximal end of the housing. The attachment feature has a base, a neck, and a button. The attachment feature includes a locking channel extending longitudinally through the button and the neck. The locking channel tapers inward in a distal direction through the button. The attachment feature includes a lateral slot extending lateral to the locking channel through the base, the neck, and the button.

A biostimulator system including the biostimulator mounted on the biostimulator transport system is also described.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 illustrates a diagrammatic cross section of a patient heart illustrating an example implantation of a biostimulator in a target anatomy, in accordance with an embodiment.
FIG. 2 is a side view of a biostimulator, in accordance with an embodiment.
FIG. 3 is a perspective view of a biostimulator transport system, in accordance with an embodiment.
FIG. 4 illustrates a perspective view of a biostimulator system having a biostimulator transport system in an unreleased state, in accordance with an embodiment.
FIG. 5 illustrates a perspective view of a biostimulator mounted on a biostimulator transport system in an unreleased state, in accordance with an embodiment.
FIG. 6 illustrates a perspective view of a biostimulator mounted on a biostimulator transport system in a released state, in accordance with an embodiment.
FIG. 7 illustrates a perspective view of a biostimulator system having a biostimulator transport system in a released state, in accordance with an embodiment.
FIG. 8 illustrates a perspective view of a biostimulator system having a biostimulator transport system in an unreleased state, in accordance with an embodiment.
FIG. 9 illustrates a perspective view of a biostimulator system having a biostimulator transport system in a released state, in accordance with an embodiment.
FIG. 10 illustrates a perspective view of a biostimulator system having a biostimulator transport system in an unreleased state, in accordance with an embodiment.
FIG. 11 illustrates a perspective view of a biostimulator system having a biostimulator transport system in an unreleased state, in accordance with an embodiment.
FIG. 12 illustrates a perspective view of a biostimulator system having a biostimulator transport system in a released state, in accordance with an embodiment.
FIG. 13 illustrates a perspective view of a biostimulator system having a biostimulator transport system in an unreleased state, in accordance with an embodiment.
FIG. 14 illustrates a perspective view of a biostimulator system having a biostimulator transport system in an unreleased state, in accordance with an embodiment.
FIG. 15 illustrates a perspective view of a biostimulator system having a biostimulator transport system transitioning toward a released state, in accordance with an embodiment.
FIG. 16 illustrates a perspective view of a biostimulator system having a biostimulator transport system in a released state, in accordance with an embodiment.
FIG. 17 illustrates a perspective view of a biostimulator system having a biostimulator transport system in an unreleased state, in accordance with an embodiment.
FIG. 18 illustrates a perspective view of a biostimulator system having a biostimulator transport system in an unreleased state, in accordance with an embodiment.
FIG. 19 illustrates a perspective view of a biostimulator system having a biostimulator transport system in a released state, in accordance with an embodiment.
FIG. 20 illustrates a perspective view of a docking cap of a biostimulator transport system having a release mechanism, in accordance with an embodiment.
FIG. 21 illustrates a perspective view of a docking cap of a biostimulator transport system having a release mechanism, in accordance with an embodiment.
FIG. 22 illustrates a perspective view of a docking cap of a biostimulator transport system having a release mechanism, in accordance with an embodiment.
FIG. 23 illustrates a perspective view of a docking cap of a biostimulator transport system having a release mechanism, in accordance with an embodiment.
FIG. 24 illustrates a perspective view of a docking cap of a biostimulator transport system having a release mechanism, in accordance with an embodiment.
FIG. 25 illustrates a perspective view of a docking cap of a biostimulator transport system having a release mechanism, in accordance with an embodiment.
FIG. 26 illustrates a perspective view of a biostimulator transport system in an unreleased state, in accordance with an embodiment.
FIG. 27 illustrates a perspective view of a biostimulator transport system in an unreleased state, in accordance with an embodiment.
FIG. 28 illustrates a perspective view of an attachment feature of a biostimulator, in accordance with an embodiment.
FIG. 29 illustrates a perspective view of an attachment feature of a biostimulator, in accordance with an embodiment.
FIG. 30 illustrates a perspective view of a docking cap of a biostimulator transport system, in accordance with an embodiment.
FIG. 31 illustrates a height comparison of various attachment features of a biostimulator, in accordance with an embodiment.
FIG. 32 illustrates a perspective view of an attachment feature of a biostimulator, in accordance with an embodiment.
FIG. 33 illustrates a perspective view of a docking cap of a biostimulator transport system, in accordance with an embodiment.

### DETAILED DESCRIPTION

Embodiments describe a biostimulator system including a biostimulator for pacing, e.g., septal pacing. The biostimulator system may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a component of a biostimulator or a biostimulator transport system. Similarly, "proximal" may indicate a second direction opposite to the first direction. Such terms are provided to establish relative frames of reference, however, and are not intended to limit the use or orientation of a biostimulator or a biostimulator transport system to a specific configuration described in the various embodiments below.

In an aspect, a biostimulator transport system incorporates a release mechanism to attach to a biostimulator in an unreleased state and to detach from the biostimulator in a released state. The release mechanism can include a tether to attach to an attachment feature of the biostimulator. The tether can be held in place in the unreleased state by a locking component, such as a locking tube or a release pin. In the released state, the locking component can be moved to a position that allows the tether to detach from the attachment feature. Accordingly, the biostimulator can be released at a target site. The various release mechanisms described below can be integrated at a distal end of the biostimulator transport system, and can require minimal travel of the locking component to achieve release, which can improve ease of release and reduce complications of delivery. More particularly, the release mechanisms are simple, inexpensive, and reliable.

In an aspect, the biostimulator includes several clutch elements around an outer shoulder of the attachment feature. The clutch elements can engage or be engaged by corresponding clutch elements of the biostimulator transport system. For example, the clutch elements can include dogs to engage slots in a docking cap of the biostimulator transport system, or slots to receive dogs of the docking cap. In any case, the clutch elements can securely engage and distribute torque evenly about the attachment feature. The distributed engagement can, in an embodiment, allow for a lower profile height than alternative attachment feature designs. Furthermore, the distributed engagement can reduce a likelihood of jamming between the attachment feature and the docking cap during retrieval or delivery. Accordingly, the biostimulator transport system and the biostimulator can benefit from torque transmission elements that have minimal length and evenly distribute torque around an attachment feature of the biostimulator.

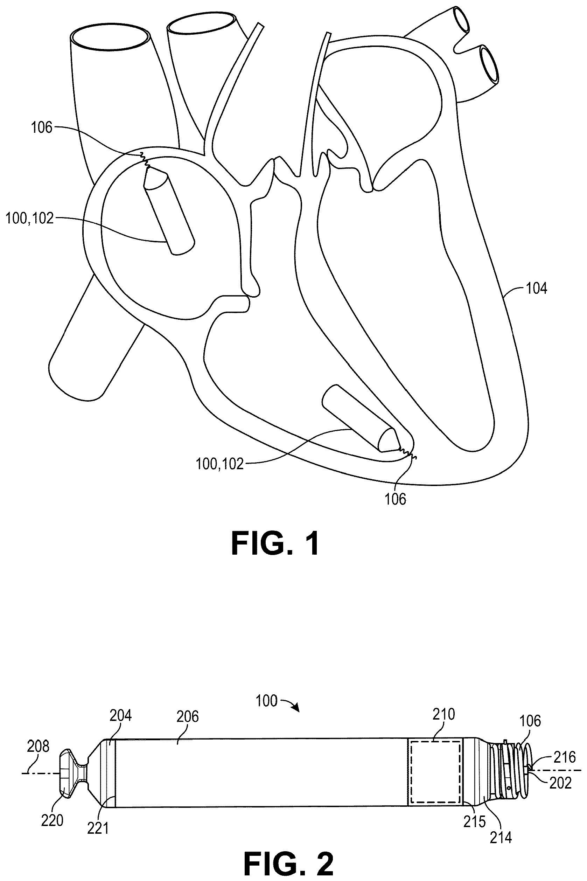

Referring to FIG. 1, a diagrammatic cross section of a patient heart illustrating an example implantation of a biostimulator in a target anatomy is shown in accordance with an embodiment. A cardiac pacing system includes one or more biostimulators 100. The biostimulators 100 can be implanted in the patient heart 104, and can be leadless, and thus may be leadless cardiac pacemakers 102. Each biostimulator 100 can be placed in a cardiac chamber, such as a right atrium and/or a right ventricle of the patient heart 104, or attached to an inside or outside of the cardiac chamber. Attachment of the biostimulator 100 to the cardiac tissue can be accomplished via one or more fixation elements 106. The fixation element(s) 106 can include a helical anchor or tines, for example. In a particular embodiment, the leadless cardiac pacemaker 102 can use two or more electrodes located on or within a housing of the leadless cardiac pacemaker 102 for pacing the cardiac chamber upon receiving a triggering signal from internal circuitry and/or from at least one other device within the body.

Referring to FIG. 2, a side view of a biostimulator is shown in accordance with an embodiment. The biostimulator 100 can be a leadless cardiac pacemaker 102 that can perform cardiac pacing and that has many of the advantages of conventional cardiac pacemakers while extending performance, functionality, and operating characteristics. The biostimulator 100 can have two or more electrodes, e.g., a distal electrode 202 and a proximal electrode 204, located within, on, or near a biostimulator housing 206 of the biostimulator 100. In an embodiment, one or more of the fixation elements 106 form a portion of the distal electrode 202. The electrodes can deliver pacing pulses to muscle of the cardiac chamber, and optionally, can sense electrical activity from the muscle. The electrodes may also communicate bidirectionally with at least one other device within or outside the body.

In an embodiment, the biostimulator housing 206 has a longitudinal axis 208, and the distal electrode 202 can be a distal pacing electrode mounted on the biostimulator housing 206 along the longitudinal axis 208. The biostimulator housing 206 can contain a primary battery to provide power for pacing, sensing, and communication, which may include, for example bidirectional communication. The biostimulator housing 206 can optionally contain an electronics compartment 210 to hold circuitry adapted for different functionality. For example, the electronics compartment 210 can be located on a central axis of the biostimulator 100 and can contain circuits for sensing cardiac activity from the electrodes, circuits for receiving information from at least one other device via the electrodes, circuits for generating pacing pulses for delivery via the electrodes, or other circuitry. The electronics compartment 210 may contain circuits for transmitting information to at least one other device via the electrodes and can optionally contain circuits for monitoring device health. The circuit of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

Leadless pacemakers or other leadless biostimulators 100 can be fixed to an intracardial implant site by one or more actively engaging mechanism or fixation mechanism. The fixation mechanism can include a screw or helical member that screws into the myocardium. Alternatively, the fixation mechanism can include tines that pierce and grip the myocardium.

In an embodiment, the biostimulator 100 includes the fixation element 106 coupled to the biostimulator housing 206. The fixation element 106 can be a helical element to screw into target tissue. More particularly, the fixation element 106 can extend helically from a flange 214 of the biostimulator 100, which is mounted on the biostimulator housing 206, to a distal tip at a helix distal end 216. Accordingly, the fixation element 106 can be coupled to a distal end 215 of the housing 206.

The helix distal end 216 can be located distal to the distal electrode 202 (a centrally located electrode). Accordingly, when the biostimulator 100 contacts the target tissue, a tip of the distal end 216 can pierce the tissue and the housing 206 can be rotated to screw the outer fixation element 106 into the target tissue to pull the distal electrode 202 into contact with the tissue.

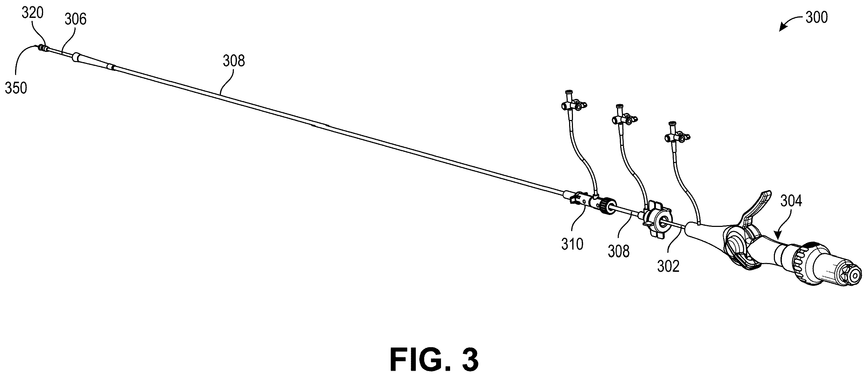

The biostimulator 100 includes an attachment feature 220. The attachment feature 220 generally facilitates coupling of the biostimulator to a biostimulator transport system (FIG. 3). More particularly, the attachment feature 220 can include features that can be engaged and retained by a release mechanism of the biostimulator transport system, as described below. In an embodiment, the attachment feature 220 is coupled to a proximal end 221 of the housing 206. For example, the attachment feature 220 can be welded to the proximal end 221 to connect the attachment feature 220 to the housing 206 physically and/or electrically.

Referring to FIG. 3, a perspective view of a biostimulator transport system is shown in accordance with an embodiment. Leadless pacemakers or other leadless biostimulators 100 can be delivered to and retrieved from a patient anatomy using a biostimulator transport system 300. In some implementations, the biostimulator transport system 300 is a delivery system for delivering the leadless pacemaker to the target tissue. In some implementations, the biostimulator transport system 300 is a retrieval system for retrieving the leadless pacemaker from the target tissue. As described below, the biostimulator transport system 300 can include a release mechanism to retain the biostimulator 100 in an unreleased state and to transition into a released state to release the biostimulator into the target anatomy.

The biostimulator transport system 300 can include an elongated catheter 302 extending distally from a handle 304 to a distal catheter end. The elongated catheter 302 can be a deflectable catheter, and an operator can use the handle 304 to steer a distal catheter end in the patient. In an embodiment, the biostimulator transport system 300 includes a guide catheter 308 mounted on the elongated catheter 302. The guide catheter 308 can be slidably disposed on the elongated catheter 302 such that a distal portion of the guide catheter 308 can slide distally over the distal catheter end of the elongated catheter 302 and/or the biostimulator 100, which may be mounted on the distal catheter end (not shown). Similarly, the biostimulator transport system 300 can include an introducer hub assembly 310 mounted on the guide catheter 308. The introducer hub assembly 310 can be slidably disposed on the guide catheter 308 such that a distal portion of the introducer hub assembly 310 can slide distally over the distal catheter end of the elongated catheter 302 and/or the distal portion of the guide catheter 308. More particularly, the introducer hub assembly 310 can be inserted into an access sheath to gain access to the patient vasculature, and after access is established, the distal portion of the guide catheter 308 and/or the distal catheter end of the elongated catheter 302 can be advanced through the access sheath into the patient.

The distal catheter end of the elongated catheter 302 may be selectively connectable to the biostimulator 100. More particularly, the biostimulator 100 can be mounted on the distal catheter end of the elongated catheter 302. The biostimulator 100 can be protected by a protective sheath of the distal portion of the guide catheter 308 during delivery and/or retrieval of the biostimulator 100 from the patient. Accordingly, the biostimulator 100 can be advanced into the patient along with the distal catheter end.

The leadless pacemaker system can be used to implant one or more biostimulators 100 within an atrium and/or a ventricle of a heart 104 of the patient. Implantation of each biostimulator 100 may be achieved, in part, by endocardial insertion of the biostimulators 100. For example, the elongated catheter 302 of the leadless pacemaker system can include a torque shaft, within an outer member 306 of the elongated catheter 302, coupled to a docking cap 320. The docking cap 320 can have a docking cavity to receive the attachment feature 220 of the biostimulator 100. The torque shaft can be torqueable and rotation of the torque shaft can rotate the docking cap 320, which can impart rotation to the attachment feature 220. Accordingly, torque can be transmitted through the torque shaft to rotate the biostimulator 100 in a first direction, e.g., clockwise. Rotating the biostimulator 100 when the fixation element 106 is in contact with the heart tissue can cause the fixation element 106 to screw into the heart tissue and affix the biostimulator 100 to the heart tissue. Similarly, removal and retrieval of the biostimulators 100 may be accomplished endocardially. For example, the torque shaft of the elongated catheter 302 can be rotated in a second direction, e.g., counterclockwise, to transmit torque through the docking cap 320 to the attachment feature 220 to disengage the biostimulator 100 from the heart tissue.

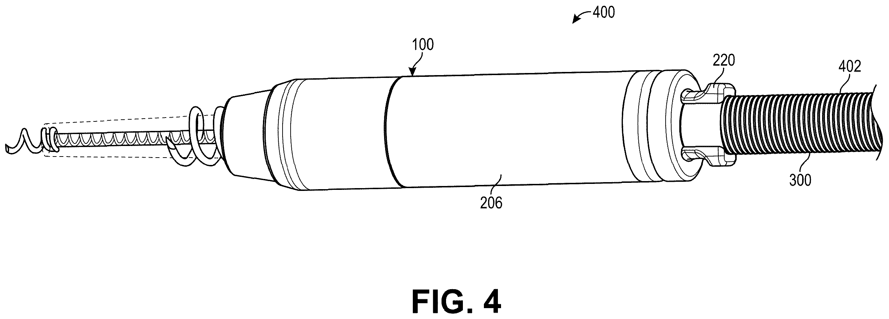

The biostimulator transport system 300 can include a release mechanism 350. The release mechanism 350 may be located at the distal catheter end. For example, the release mechanism can include a locking tube and tether (FIGS. 4-9) that extend through a shaft lumen of the torque shaft to a terminal end distal to the docking cap 320. The release mechanism can engage the biostimulator 100 in the unreleased state and disengage from the biostimulator 100 in the released state. Accordingly, delivery and retrieval systems having a structure similar to that shown in FIG. 3 may be used to deliver and/or retrieve the biostimulator 100 from a target anatomy.

Referring to FIG. 4, a perspective view of a biostimulator system having a biostimulator transport system in an unreleased state is shown in accordance with an embodiment. A biostimulator system 400 can include the biostimulator transport system 300 and the biostimulator 100. For example, the biostimulator 100 may be mounted on, e.g., at a distal end of, the biostimulator transport system 300. In an embodiment, the biostimulator transport system 300 includes a locking tube 402, and the biostimulator 100 is mounted on the biostimulator transport system 300 at a distal end of the locking tube 402. For example, the distal end of the locking tube 402 can engage the attachment feature 220 of the biostimulator 100.

The locking tube 402 can releasably engage the attachment feature 220, as described below. More particularly, the locking tube 402 can actuate between an unreleased state and a released state to capture or release the biostimulator 100. The locking tube 402 may interact with a corresponding component of the biostimulator transport system 300, such as a locking tether and/or ball to cause the system to grip or release the biostimulator 100.

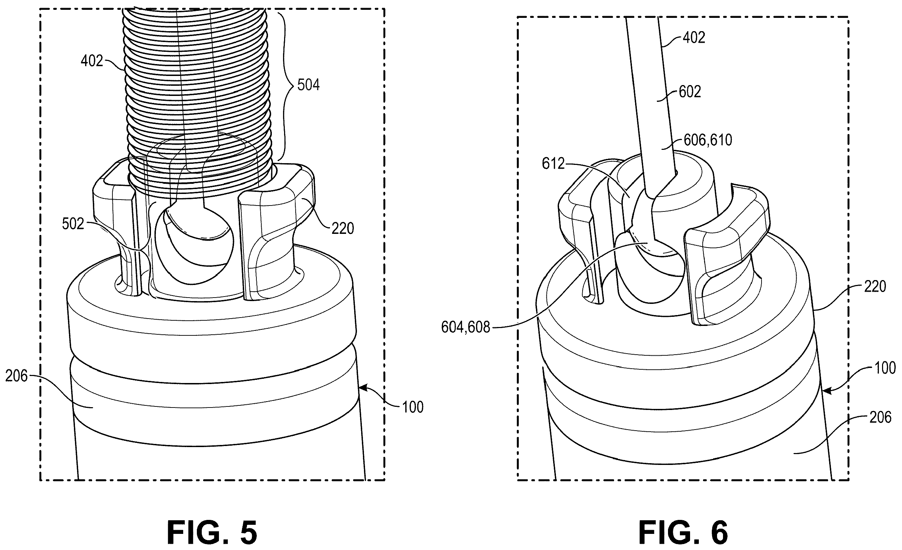

Referring to FIG. 5, a perspective view of a biostimulator mounted on a biostimulator transport system is shown in accordance with an embodiment. The locking tube 402 can include a tubular component having a central lumen. For example, the locking tube 402 can include a distal tube section 502 and a proximal tube section 504, and the central lumen can extend through the tube sections along the central axis. Accordingly, another component, such as a tether described below, may be moved through the central lumen, interior of the locking tube 402.

The locking tube 402 may fit partially within the attachment feature 220. As shown, the attachment feature 220 can include laterally separated feet extending proximally, e.g., upward, on either side of the locking tube 402. The feet can have radially extending toes that can be gripped, for example, by a retrieval snare. More particularly, the retrieval snare can lasso the feet to grip and retract the biostimulator 100. Such retraction may occur during retrieval of the biostimulator 100, after the locking tube 402 has already been released from the biostimulator 100.

In an embodiment, the distal tube section 502 is stiffer than the proximal tube section 504. More particularly, the proximal tube section 504, which may be proximal to the attachment feature 220, may be more flexible than the distal tube section 502, which can coincide with the attachment feature 220 when the biostimulator transport system 300 engages the biostimulator 100. The distal tube section 502 can include a solid hypotube section at the most distal tip of the locking tube 402. A solid wall of the hypotube section can be rigid. The distal tube section 502 may fit within the recess formed between the proximally-extending feet of the attachment feature 220. Accordingly, the rigid distal tube section 502 can firmly hold the attachment feature 220 at a location radially inward from the feet.

In contrast to the distal tube section 502, the proximal tube section 504 may be flexible. For example, the proximal tube section 504 can include a flexible polymeric or metallic braid or coil. In an embodiment, the proximal tube section 504 includes a closed pitch coil. For example, the closed pitch coil may be formed from a hypotube having a spiral cut. The closed pitch coil may be flexible enough to move with the beating of the heart 104 when the biostimulator 100 is implanted at a target tissue site. More particularly, the proximal tube section 504 can flex under the pulsatile contractions of the heart 104, without transmitting significant forces to the biostimulator 100 that could cause the biostimulator to dislodge from the heart tissue.

The distal tube section 502 may be rigid to retain a tether (FIG. 6) within the attachment feature 220. More particularly, the locking tube 402 can constrain the tether within the attachment feature 220. The tether can interfere with the attachment feature 220 to connect the biostimulator transport system to the biostimulator 100. Actuation of the locking tube 402, e.g., retracting the locking tube 402, can move the distal tube section 502 away from the attachment feature 220 to transition the biostimulator transport system 300 from an attached state to a detached state, as described below.

Referring to FIG. 6, a perspective view of a biostimulator mounted on a biostimulator transport system is shown in accordance with an embodiment. The biostimulator transport system 300 can include a tether 602. The tether 602 can engage the attachment feature 220 of the biostimulator 100 to lock the biostimulator transport system 300 to the biostimulator 100. Engagement of the attachment feature 220 can include insertion of the tether 602 into a locking slot of the attachment feature 220. The locking slot can include, for example, a transverse hole within which a portion of the tether 602 fits.

In an embodiment, the tether 602 extends through the central lumen of the locking tube 402 (FIG. 5). The tether 602 can include a locking end 604 coupled to a tether body 606. For example, the locking end 604 may include an enlarged component, relative to the tether body 606. The enlarged component can include a spherical component, e.g., a locking ball 608, attached to a distal end of the tether body 606. The locking end 604 can fit into a cavity, such as the locking slot, formed in the attachment feature 220. For example, the cavity can be a hole that is sized and shaped to receive the locking ball 608. The cavity may, for example, include a cylindrical hole having a diameter slightly larger than a diameter of the locking ball 608. The locking slot can extend laterally into the attachment feature 220 to allow the locking ball 608 to insert into the hold and slide laterally inward into the prong of the attachment feature 220 that extend proximally between the snare feet. The cylindrical hole can have a diameter greater than the locking end 604 to allow the locking end 604 to be received within the cavity.

The locking end 604 may be a rigid and/or resilient member that engages and interferes with the surfaces surrounding the cavity in the attachment feature 220. By contrast, the tether body 606 may include a fiber 610 that is very flexible. For example, the fiber 610 may include a polymeric filament or a metallic filament that is elongated and flexible. In an embodiment, the fiber 610 is one of several fibers making up a cable. More particularly, the tether body 606 may include an elongated, flexible cable.

The locking end 604 can be coupled to the tether body 606 of the tether 602. For example, the locking end 604 and the tether body 606 may be formed from similar materials, such as a same metal, and the locking end 604 may be welded or soldered to the tether body 606. Alternatively, the locking end 604 may be formed from a different material than the tether body 606, and an alternative bond may be used, such as an adhesive bond, to attach the locking end 604 to the tether body 606. The locking end 604 can alternatively be integrally formed with the tether body 606.

In an embodiment, as described below, the attachment feature 220 can include a locking channel 612 extending through the attachment feature 220 into the cavity. For example, the locking channel 612 can extend longitudinally through a proximal face of the prong that contains the locking slot. Whereas the locking channel 612 may be sized to receive the tether body 606, the cavity may be sized to receive the locking end 604. More particularly, the locking end 604 can be wider than the tether body 606 and, thus, the cavity that provides the locking slot to receive the locking end 604 may be wider than the locking channel 612 that is sized to receive the tether body 606. The locking end 604, e.g., the locking ball 608, can fit into the cavity that is formed in a side of the attachment feature 220. Similarly, the tether body 606 can fit into the locking channel 612. The locking end 604 may, however, be too large to pass into the locking channel 612. The locking end 604 can therefore interfere with the attachment feature 220 such that, when the locking end 604 is in the cavity, the tether 602 cannot be pulled away from the attachment feature 220. The locking end 604 can seat in the cavity and prevent the locking end 604 from exiting through the locking channel 612 to release the biostimulator 100.

A radial gap can be located between the snare feet and the prong of the attachment feature 220. The radial gap may be sized to allow the locking tube 402 to advance forward into the attachment feature 220, between the proximally-extending components. More particularly, the tubular structure of the locking tube 402 and, in particular, the distal tube section 502 can have a wall thickness that is less than the radial gap. The distal tube section can therefore slide into the radial gap to surround the locking end 604 that rests within the locking slot in the prong.

Referring again to FIG. 5, when the locking tube 402 is advanced over the tether 602 such that the distal tube section 502 surrounds the cavity in the attachment feature 220, the wall of the distal tube section 502 can confine the locking end 604 within the cavity. For example, lateral movement of the locking end 604 out of the cavity can be prevented by the surrounding distal tube section 502. The locking tube 402 can therefore prevent any spontaneous release of the locking end 604 from the attachment feature 220. The locking tube 402, when advanced, therefore locks the tether 602 to the biostimulator 100. The substantial flexibility of the proximal tube section 504 and the tether body 606 allows the biostimulator 100 to move freely within the heart chamber. For example, in a tether mode, the biostimulator 100 can be attached to the target tissue and the docking cap 320 of the biostimulator transport system 300 can be retracted. The biostimulator 100 may be connected to the biostimulator transport system 300, therefore, by a length of the tether 602 between the biostimulator 100 and the docking cap 320. The flexibility of the length can allow the biostimulator 100 to move freely while the heart pulses. Accordingly, a physician can assess whether the biostimulator 100 is properly affixed to the target tissue.

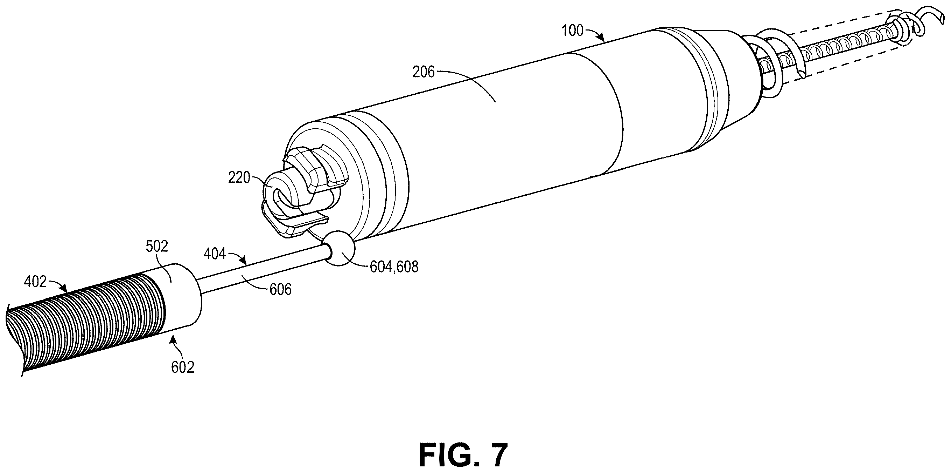

Referring to FIG. 7, a perspective view of a biostimulator system having a biostimulator transport system in a released state is shown in accordance with an embodiment. When proper affixation of the biostimulator 100 to the target tissue has been confirmed in the tether mode, the tether 602 may be actuated to release the biostimulator 100. In an embodiment, the tether 602 is actuated by retracting the locking tube 402 relative to the tether body 606. Retraction can pull the distal tube section 502 out of the radial gap of the attachment feature 220. For example, the handle 304 may be coupled to the locking tube 402 and the tether 602 to provide relative movement between the distal tube section 502 of the locking tube 402 and the locking end 604 of the tether 602. A grip of the handle may be fixed to the tether 602, and a sliding portion of the handle that is movable relative to the grip can be fixed to the locking tube 402. The sliding portion may be actuated to move the locking tube 402 relative to the tether 602. Movement of the locking tube 402 relative to the tether body 606 can transition the biostimulator transport system 300 from an attached (or unreleased) state to a detached state. In the attached state, the distal tube section 502 can coincide with and/or surround the attachment feature 220. The handle 304 can be manipulated to cause the distal tube section 502 to retract proximal to the attachment feature 220 to transition into the detached state. More particularly, the distal tube section 502 may be retracted to expose the cavity laterally from the attachment feature 220. When the cavity is exposed in the detached (or released) state, the locking end 604, e.g., the locking ball 608, can exit the cavity. The movement of the heart 104 can urge the locking ball 608 to move out of the slot in the attachment feature 220. Similarly, the tether body 606 can move laterally out of the locking channel 612. Thus, the tether 602 can detach from the biostimulator 100, leaving the biostimulator 100 in the heart chamber at the target site.

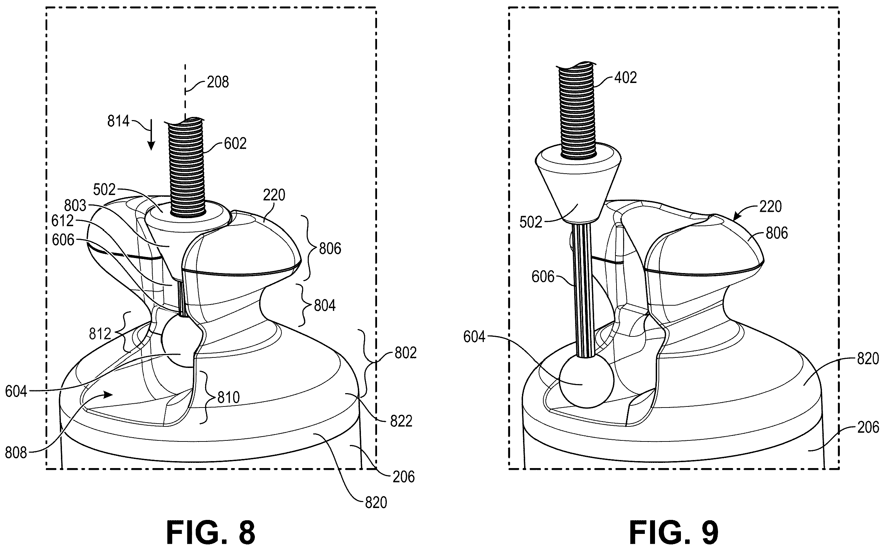

Referring to FIG. 8, a perspective view of a biostimulator system having a biostimulator transport system in an unreleased state is shown in accordance with an embodiment. Rather than surrounding the locking channel 612 within which the tether 602 is engaged, the distal tube section 502 may insert into the channel. In an embodiment, the distal tube section 502 has a conical profile 803. More particularly, a proximal end of the distal tube section 502 can be wider than a distal end of the tube section. The conical profile 803 can therefore taper from the proximal end to the distal end. Similarly, the locking channel 612 may have a tapered section to receive the distal tube section 502. For example, the conical feature can be tapered to engage the tapered section such that an outward facing conical surface of the locking tube 402 opposes an inward facing surface surrounding the locking channel 612. Accordingly, the conical profile 803 of the distal tube section 502 can support and stabilize the locking tube 402 relative to the attachment feature 220 when the distal tube section 502 is inserted into the locking channel 612.

The attachment feature 220 may be segmented into portions. The portions can be arranged along the longitudinal axis 208 of the attachment feature 220. In an embodiment, the portions of the attachment feature 220 include a base 802, a neck 804, and a button 806. Such segments may, however, be segmented for purposes of description. For example, the button 806 may be a proximal most portion of the attachment feature 220, the base 802 may be a distal most portion of the attachment feature 220, and the neck 804 may be a portion of the attachment feature 220 intermediate between the button 806 and the base 802, and having a cross-sectional dimension that is smaller than both the button 806 and the base 802.

The base 802 may mount on a proximal end of the housing 206. Accordingly, the base 802 may have an outer dimension, e.g., at a proximal lip 820 that is equal to an outer dimension of the housing 206. By contrast, the neck 804 may be narrower than the base 802. For example, the base 802 can taper inward from a distal most edge of the lip 820 at the housing 206 to a proximal most edge at the neck 804. The button 806 may extend proximally from the neck 804 and taper outward to a larger dimension. For example, the button 806 may include an anvil shape that widens to allow a retrieval snare to grasp the attachment feature 220 around the neck 804 distal to the button 806.

The base 802 can include an outer shoulder 822 extending about the longitudinal axis 208 of the attachment feature 220. For example, the outer shoulder 822 can include an edge, e.g., a radiused edge, transitioning from the lip 820 of the base 802 that mounts on the housing 206 to the tapered surface of the base 802 that leads to the neck 804. As described below, the outer shoulder 822 can have one or more torque transmission features, e.g., clutch elements, that allow torque to be uniformly transferred to the attachment feature 220 to drive the biostimulator into the target tissue. More particularly, the outer shoulder 822 can be engaged by the docking cap 320 to transfer torque from the biostimulator transport system 300 to the biostimulator 100.

The locking channel 612 can extend longitudinally through the button 806. The locking channel 612 may therefore receive the tether 602 along the central axis of the biostimulator 100. In an embodiment, the attachment feature 220 includes the cavity, as in FIGS. 5-7, to receive the locking end 604 laterally through the attachment feature 220. The lateral slot 808 can extend laterally to the locking channel 612 through the base 802, the neck 804, and the button 806. More particularly, the locking channel 612 can be a laterally open channel exposed to the surrounding environment radially outward from the central axis. Accordingly, when the locking tube 402 is retracted to provide a gap, between the locking end 604 and the distal tube section 502, which is greater than a length of the button 806 and neck 804 together, the locking end 604 can slide laterally through the locking slot into (or out of) the locking channel 612 (FIG. 9).

The locking slot, which provides lateral access to the locking channel 612 through the attachment feature 220, can have portions sized to receive the tether 602. In an embodiment, a base portion 810 of the lateral slot 808 may extend laterally through the base 802. The base portion 810 may be wider than a neck portion 812 of the lateral slot 808 that extends through the neck 804 of the attachment feature 220. The difference in widths can allow the base portion 810 to receive a wider portion of the tether 602 than the neck portion 812. More particularly, the base portion 810 can be sized to receive the locking end 604 of the tether 602, and the neck portion 812 can be sized to receive the tether body 606 of the tether 602.

The locking channel 612 can include a segment sized to receive the distal tube end, as described above. More particularly, the locking channel 612 may be wider within the button 806 than within the neck 804. The locking channel 612 within the button 806 can taper inward in a distal direction 814 through the button 806. For example, the tapered channel segment can match the conical profile 803. Accordingly, when the locking end 604 is in the base portion 810 of the lateral slot 808, the tether body 606 is inserted into the neck portion 812 of the locking channel 612, and the conical portion is advanced in the distal direction 814, the button 806 can be sandwiched between the distal tube section 502 and the locking end 604. The locking end 604 can be located in the locking channel 612 in the neck 804 and in a proximal length of the button 806, and the tether 602 can be located in the locking channel 612 in the button 806. The tether 602 can squeeze the button 806 and, because the locking end 604 is adjacent to the narrower segment of the locking channel 612 in the neck 804, the locking end 604 can be confined to the locking channel 612.

The biostimulator transport system 300 may include a mechanism to bias the locking tube 402 in the distal direction 814 when in the unreleased state. For example, the biostimulator transport system 300 can include a compression member (not shown) to bias the locking tube 402 distally relative to the tether body 606. The compression member can include a spring or a compression mechanism that applies a forward pressure to the locking tube 402 relative to the tether body 606. In the embodiments described above, when the locking tube 402 is biased distally, the locking tube 402 can lock the locking end 604 into the locking channel 612. When the locking end 604 is confined within the locking channel 612, a likelihood of detachment of the biostimulator 100 from the tether 602 is reduced. The compression member can bias the locking tube 402 into or around the attachment feature 220, and can bias the locking end 604 into a section of the locking channel 612 that the locking end 604 cannot exit when the locking tube 402 is biased distally. Accordingly, the compression member can bias the biostimulator transport system 300 into the attached state until a user wants to transition the system into the detached state to release the biostimulator 100.

Referring to FIG. 9, a perspective view of a biostimulator system having a biostimulator transport system in a released state is shown in accordance with an embodiment. Releasing the tether 602 having the tapered distal tube section 502 from the attachment feature 220 can occur in a similar fashion to that described above. More particularly, the locking tube 402 can be retracted relative to the locking end 604 to unwedge the distal tube section 502 from the button 806 of the attachment feature 220. As the gap between the locking end 604 and the distal tube section 502 increases, the locking end 604 can move distally within the locking channel 612 to align with the base portion 810 of the lateral slot 808. Movement of the biostimulator 100 can urge the locking end 604 outward through the wider base portion 810 of the lateral slot 808. Accordingly, the biostimulator 100 can detach from the tether 602 to remain at the target site. The biostimulator transport system 300 may, by contrast, be retrieved from the patient anatomy.

Removed from the locking cannel 612, a shape of the distal tube section 502 is more readily visible. The shape can have an outer surface that tapers inward in a distal direction from a proximal face of the conical insert to a distal end of the conical insert. The insert, e.g., the conical component that inserts into the locking channel 612, can be mounted on a distal portion of the locking tube 402. For example, the flexible locking tube can have a distal end, and the conical insert, which may be a solid component having the tapered outer surface, can be welded, glued, or otherwise adhered to the distal end. The tether body 606, by contrast, can slide axially through a central lumen of the locking tube 402 to allow the tether ball 604 to approach or retreat from the tapered insert. More particularly, the ball and tether can be separated by a changeable gap that, when minimized by retracting the tether body 606, can pinch the attachment feature 220 to lock the biostimulator 100 to the biostimulator transport system 300.

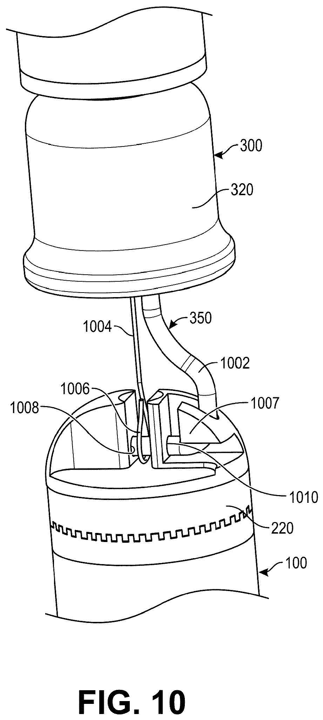

Referring to FIG. 10, a perspective view of a biostimulator system having a biostimulator transport system in an unreleased state is shown in accordance with an embodiment. The biostimulator transport system 300 can include a release mechanism 350 to connect to the biostimulator 100 using a wire lock concept. As shown, the release mechanism 350 can connect the attachment feature 220 of the biostimulator 100 to a distal end of the biostimulator transport system 300.

In an embodiment, the wire lock 1002 concept includes a wire lock to act as an anchor to capture and hold a fiber tether 1004. The wire lock 1002 can be a length of wire, e.g., a nickel-titanium wire. Alternatively, the wire lock 1002 can have a hybrid structure including a segment of metallic wire to engage the fiber tether 1004, and a segment of polymeric fiber attached to an end of the metallic segment. In any case, the wire lock 1002 can engage and constrain the fiber tether 1004, as described below.

The fiber tether 1004, like the tether body 606 described above, may include a flexible fiber. The fiber tether 1004 can have a loop 1006 at a distal end. The loop 1006, when constrained relative to the biostimulator 100, can hold the tether 602 in place relative to the attachment feature 220. The fiber tether 1004 may be connected to the biostimulator transport system 300, e.g., at the handle 304. Accordingly, retention of the loop 1006 of the fiber tether 1004 at the attachment feature 220 can interconnect the biostimulator 100 and the biostimulator transport system 300.

In an embodiment, the loop 1006 can be retained at the attachment feature 220 by the wire lock 1002. The attachment feature 220 may include a longitudinal slot within which the loop 1006 can be inserted. The slot may be formed, for example, between adjacent vertical walls of the attachment feature 220.

The wire lock 1002 can extend laterally through the slot. More particularly, the wire lock 1002 can be inserted into a cavity in the attachment feature 220, and slid through holes in vertical walls defining the slot, through the loop 1006, to hold the loop 1006 in place. The wire lock 1002 can extend across a gap of the slot, and can be constrained to each of the vertical walls. The loop 1006 may therefore be held in place by the constrained wire lock 1002.

The wire lock 1002 can be bent and slid into a cradle 1007 of the attachment feature 220. The cradle 1007 can hold the wire lock 1002 in a deformed state. Spring energy in the wire lock 1002 can bias the wire lock 1002 in a distal direction to remain engaged in the holes of the vertical walls. Accordingly, the cradle 1007 can be used to preload the wire lock 1002 in a pre-curve. The pre-curve of the wire lock 1002 can extend back into the docking cap 320 and the catheter shaft to ensure concentricity and no side loads.

Constraint of the wire lock 1002 at each of the vertical walls defining the gap of the slot may be made by passing the wire lock 1002 into holes in the vertical walls. For example, a distal end of the wire lock 1002 can insert into a first hole 1008 in a first vertical wall of the attachment feature 220, and the wire lock 1002 can extend proximally across the gap into and through a second hole 1010 in a second vertical wall of the attachment feature 220. The wire lock 1002 forms a bridge across the gap. When the loop 1006 of the fiber tether 1004 is slid over the wire lock 1002, the fiber tether 1004 is held in place and prevented from being retracted from the slot. The tether 602 is captured and the wire lock 1002 is held in place.

When the tether 602 is in the unreleased state, joining the biostimulator 100 to the biostimulator transport system 300, the biostimulator 100 can be implanted. Furthermore, the docking cap 320 can be retracted to place the system into the tether mode. When fixation of the biostimulator 100 to the target tissue is confirmed, the biostimulator 100 can be released. The biostimulator 100 may involve transitioning the biostimulator transport system 300 into the released state. To do so, a proximal load can be applied to the wire lock 1002, e.g., by a handle mechanism. Pulling on the wire lock 1002 can retract the wire lock from the first hole 1008 and the second hole 1010. More particularly, the wire lock 1002 can be pulled laterally through the loop 1006 until the distal end of the wire lock 1002 is retracted through the second hole 1010. Retraction of the wire lock 1002 may occur while the biostimulator 100 is docked in the docking cap 320. More particularly, the docking cap 320 can be advanced to capture the attachment feature 220 prior to retracting the wire lock 1002. When the wire lock 1002 is pulled proximally by the mechanism of the handle 304, the wire lock 1002 slides out of the cavity and back into the docking cap 320. The loop 1006 is released and free to exit the cavity in the attachment feature 220. More particularly, the biostimulator 100 becomes detached from the biostimulator transport system 300, and can remain at the tissue site while the biostimulator transport system 300 is retrieved from the patient's anatomy.

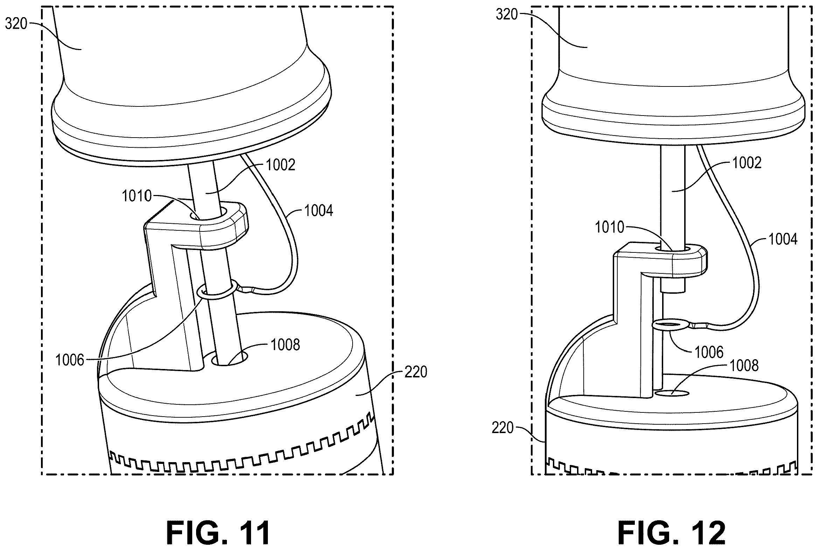

Referring to FIG. 11, a perspective view of a biostimulator system having a biostimulator transport system in a released state is shown in accordance with an embodiment. The wire lock 1002 concept can be embodied in various configurations. For example, rather than extending into the slot in an axial direction, the tether 1004 and loop 1006 may extend into the slot in a lateral direction.

As described above, the slot may be defined between walls, e.g., horizontal walls, in which a first hole 1008 and a second hole 1010 are formed. The wire lock 1002 can extend through the holes to bridge the gap of the slot. In an embodiment the wire lock 1002 extends through the holes in the axial direction. Accordingly, the wire lock 1002 does not have to be inserted into the cavity and bent. Rather, the wire lock 1002 can be an elongated, straight wire extending distally from the docking cap 320. The wire lock 1002 can extend straight through the guide holes in the horizontal walls and through the loop 1006. The straight wire can capture the loop 1006 and constrain the tether 602 relative to the attachment feature 220. When the wire is kept in a fixed position, the fiber tether 1004 is constrained, and the biostimulator 100 is tethered to the biostimulator transport system 300 in the unreleased state.

Referring to FIG. 12, a perspective view of a biostimulator system having a biostimulator transport system in an unreleased state is shown in accordance with an embodiment. Similar to the lateral wire lock, the axial wire lock 1002 can be actuated by a handle mechanism to retract from the first hole 1008. When the wire lock 1002 is retracted to a location that is proximal to the loop 1006, the loop can be released from the wire lock 1002. In the released state, the biostimulator 100 can detach from the biostimulator transport system 300 to remain at the target tissue. The biostimulator transport system 300, including the fiber tether 1004 and the wire lock 1002, can be pulled away from the biostimulator 100 and retrieved from the patient's anatomy.

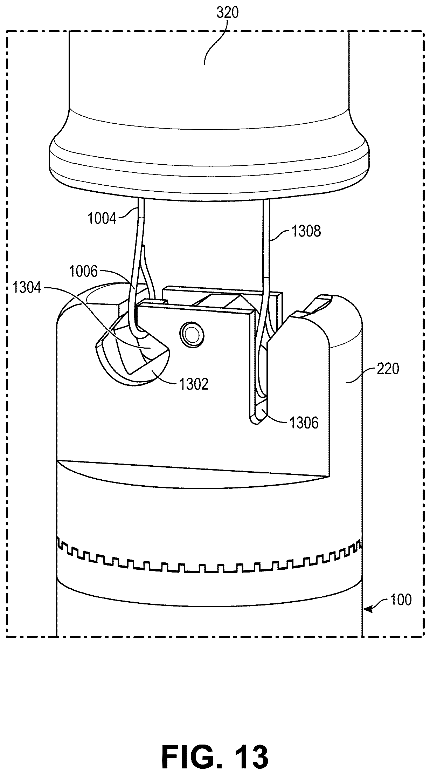

Referring to FIG. 13, a perspective view of a biostimulator system having a biostimulator transport system in an unreleased state is shown in accordance with an embodiment. The biostimulator transport system 300 can include a release mechanism to connect to the biostimulator 100 using a locking key concept. In the locking key concept, a rotating key 1302 can pivot within the attachment feature 220 of the biostimulator 100. The attachment feature 220 can include several slots, e.g., a tether slot 1304 and a locking slot 1306, sized to receive respective lengths of fibers 610. More particularly, the loop 1006 of the fiber tether 1004 can be inserted into the tether slot 1304, and the locking slot 1306 can receive a locking fiber 1308. As described above, the fiber tether 1004 can join the biostimulator 100 to the biostimulator transport system 300 and allow the system to function in the tether mode. The locking fiber 1308 can be used to hold the key 1302 in an unreleased state, as described below.

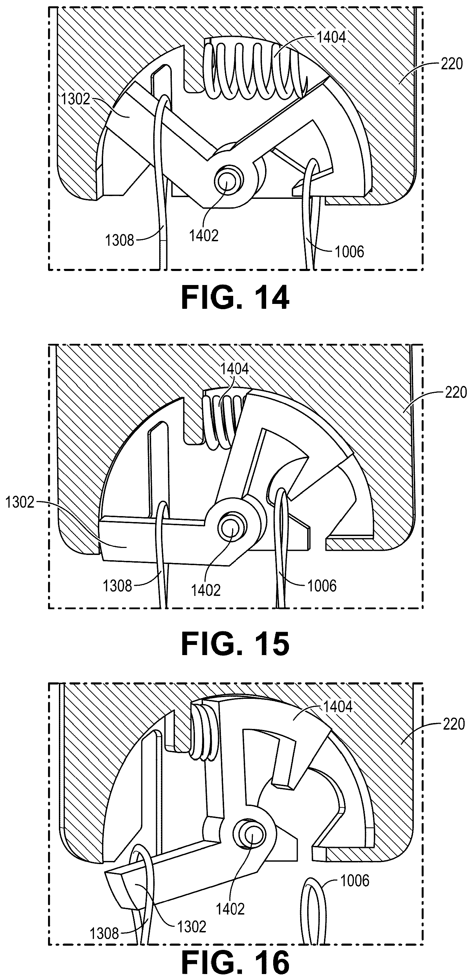

Referring to FIG. 14, a perspective view of a biostimulator system having a biostimulator transport system in an unreleased state is shown in accordance with an embodiment. The key 1302 within the attachment feature 220 of the biostimulator 100 can pivot about a pin 1402. A first portion of the key 1302 can have a hook shape. In the unreleased state, the hook shape can, in combination with a wall of the attachment feature 220, define a hole within which the fiber tether 1004 can be inserted. More particularly, the hook and the wall of the attachment feature 220 can extend through the loop 1006 of the fiber tether 1004. The second portion of the key 1302, on an opposite side of the pin 1402 from the first portion, can include a lever. The lever can, in combination with the wall of the biostimulator 100, define a hole within which the locking fiber 1308 can fit. More particularly, the lever can extend through the loop 1006 of the locking fiber 1308 to constrain the fiber 610 in a slot in the wall.

The key 1302 can be held in the unreleased state by a spring 1404. The spring 1404 can be located within a cavity in the attachment feature 220, and can bias the first portion of the key 1302, having the hook shape, toward the closed position. More particularly, the spring 1404 can provide a spring force to keep the rotating key 1302 from spontaneously releasing. In the closed position, the hook can hold the fiber tether 1004, allowing the biostimulator transport system 300 to transition to the tether mode.

Referring to FIG. 15, a perspective view of a biostimulator system having a biostimulator transport system transitioning toward a released state is shown in accordance with an embodiment. The locking fiber 1308 can be used to actuate the release mechanism to transition the biostimulator transport system 300 into the released state. The locking fiber 1308 can be pulled to rotate the key 1302 about the pin 1402. As the key 1302 rotates, the spring 1404 can compress. Furthermore, the hook can rotate proximally, creating a gap between a tip of the hook and the wall of the attachment feature 220. The gap provides a path through which the loop 1006 of the fiber tether 1004 may pass. Accordingly, in the state shown in FIG. 15, the fiber tether 1004 can be released from the biostimulator 100.

In the transitional state, the locking fiber 1308 may remain within the slot in the attachment feature 220. The lever may rotate proximally, however, it may still interfere with removal of the locking fiber 1308 from the slot. Accordingly, the fiber tether 1004 may be released from the biostimulator 100 prior to release of the locking fiber 1308.

Referring to FIG. 16, a perspective view of a biostimulator system having a biostimulator transport system in a released state is shown in accordance with an embodiment. After the fiber tether 1004 is released from the biostimulator 100, the locking fiber 1308 may be pulled further proximally. The pulling force can rotate the key 1302 until the lever rotates proximally relative to the attachment feature 220. More particularly, a gap between the lever and the attachment feature 220 can be formed. The biostimulator transport system 300 can be fully transitioned into the released state. In the released state, the locking fiber 1308 can slide out of the gap. When the locking fiber 1308 slides out of the gap, the biostimulator 100 is released from the biostimulator transport system 300. Biostimulator 100 can remain implanted at the target tissue while the biostimulator transport system 300 is retrieved from the patient anatomy.

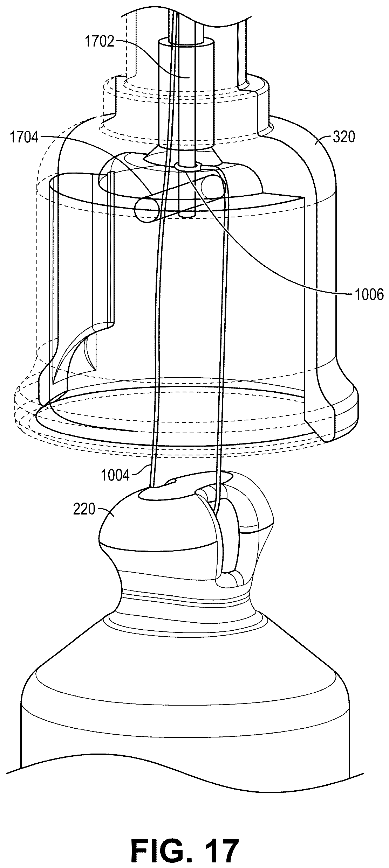

The biostimulator transport system 300 can include a release mechanism to connect to the biostimulator 100 using a crossbar release concept. The crossbar release concept may be embodied in various configurations, as described below with respect to FIGS. 17-27.

Referring to FIG. 17, a perspective view of a biostimulator system having a biostimulator transport system in an unreleased state is shown in accordance with an embodiment. The crossbar release mechanism can include several components. The components can include a release pin 1702, the fiber tether 1004, and a crossbar 1704. The several components can work together to secure the biostimulator 100 to the biostimulator transport system 300, and to release the biostimulator 100 when required. In an embodiment, the crossbar release mechanism utilizes the fiber tether 1004 to secure the biostimulator 100 to the biostimulator transport system 300. The fiber tether 1004 can extend around the crossbar 1704, and extend through the loop 1006 to retain the fiber tether 1004. More particularly, the fiber tether 1004 can be constrained around the crossbar 1704 by the release pin 1702, and the biostimulator 100 can stay secured to the biostimulator transport system 300.

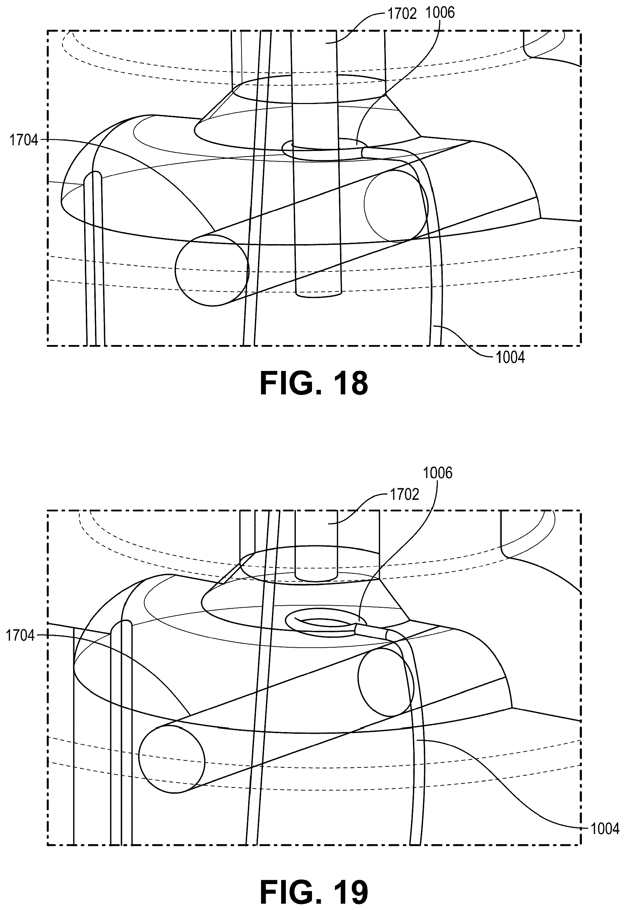

Referring to FIG. 18, a perspective view of a biostimulator system having a biostimulator transport system in an unreleased state is shown in accordance with an embodiment. The biostimulator 100 is secured when the release pin 1702 extends beyond, e.g., distal to, the crossbar 1704. When the release pin 1702 is so located, the fiber tether 1004 can be pulled against the crossbar 1704 and the crossbar can prevent the fiber tether 1004 from coming off.

Referring to FIG. 19, a perspective view of a biostimulator system having a biostimulator transport system in a released state is shown in accordance with an embodiment. Converse to the unreleased state shown in FIG. 19, the biostimulator transport system 300 can be transitioned into the released state by retracting the release pin 1702 proximal to the crossbar 1704. More particularly, the fiber tether 1004 can be coupled to a handle mechanism that pulls and retracts the release pin 1702 in the axial direction. When retracted through the loop 1006 of the fiber tether 1004, the release pin 1702 can detach from the fiber tether 1004 to release the biostimulator 100 from the biostimulator transport system 300.

The docking cap 320 can have various configurations to provide structure that performs the function of the crossbar 1704 described above. Several such configurations are described below. In each case, the release mechanism is advantageously located at the distal end of the biostimulator transport system 300. Locating the release mechanism at the distal end can have several advantages, as compared to release mechanisms located at a proximal end or in the handle 304 of the biostimulator transport system 300. For example, the effective release length of the fiber tether 1004, which may be defined as a distance the fiber tether 1004 has to travel to consistently release the biostimulator 100 from the biostimulator transport system 300 may be reduced. Release may therefore be achieved by retracting the release pin 1702 only a few millimeters. By contrast, release mechanisms located in the handle 304 can require long actuation throws, e.g., tens of inches or more, to achieve release. Accordingly, a likelihood of fibers becoming tangled during release may be reduced, and complications with dislodgment, difficulty releasing, etc. may also be reduced. The distal location of the release mechanism 350 can also reduce procedural time. More particularly, given that release of the biostimulator 100 requires short actuation throws, less time is required to move the fiber tether 1004, and the related procedure can be shortened.

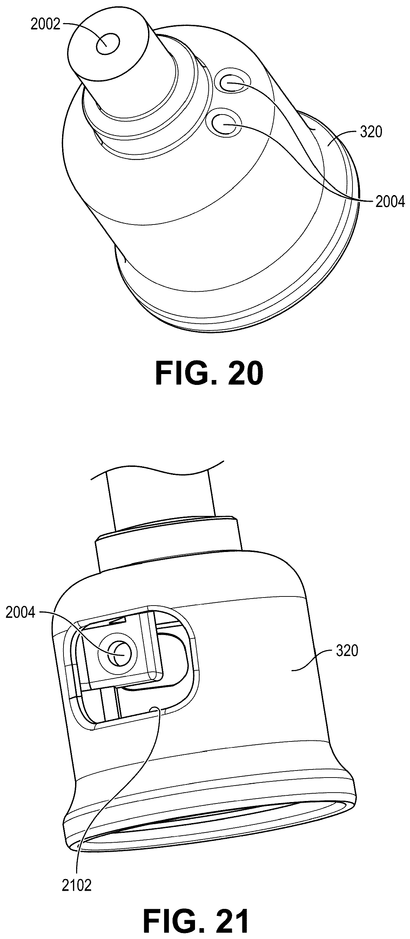

Referring to FIG. 20, a perspective view of a docking cap of a biostimulator transport system having a release mechanism is shown in accordance with an embodiment. The crossbar release mechanism can include the docking cap 320 having several holes. A pin hole 2002 can extend centrally through the docking cap 320 in the axial direction. The pin hole 2002 can receive the release pin 1702 and allow the release pin to slide distally and proximally to transition between the unreleased state and the released state.

Several tether holes 2004 can be located in a proximal wall of the docking cap 320. The tether holes 2004 can be circumferentially offset from each other along the wall, and about a central axis extending through the pin hole 2002. The fiber tether 1004 can extend distally through one or more of the tether holes 2004. The loop 1006 of the fiber tether 1004 can engage the release pin 1702 within the docking cavity of the docking cap 320, which is hidden by the docking cap wall in FIG. 20. The release pin 1702 may therefore retain and/or release the loop 1006 to couple or decouple the biostimulator 100 from the biostimulator transport system 300.

Referring to FIG. 21, a perspective view of a docking cap of a biostimulator transport system having a release mechanism is shown in accordance with an embodiment. The docking cap 320 can include one or more windows 2102 to allow an internal feature to be machined into the docking. More particularly, the docking cap 320 can include a lateral wall extending into the docking cavity. A tether hole 2004 may be located in the lateral wall. The lateral wall can fulfill the function of the crossbar 1704. More particularly, the fiber tether 1004 can be looped through the tether hole 2004, and the release pin 1702 can engage or disengage the loop 1006 of the fiber tether 1004 to retain or release the biostimulator 100 from the biostimulator transport system 300.

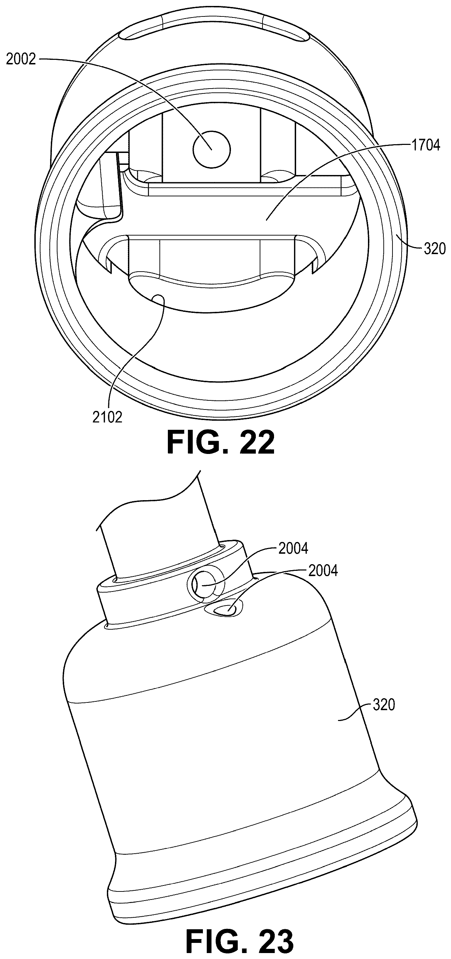

Referring to FIG. 22, a perspective view of a docking cap of a biostimulator transport system having a release mechanism is shown in accordance with an embodiment. The docking cap 320 can include the crossbar 1704 machined into the docking cavity. More particularly, the docking cap 320 can be formed as a monolithic structure having a crossmember or crossbar 1704 formed to extend laterally through the cavity that receives the attachment feature 220. The pin hole 2002 can extend axially through the docking cap 320, and can be laterally offset from the crossbar 1704. In operation, the fiber tether 1004 can loop around the crossbar 1704, to engage or disengage from the pin 1402 extending through the pin hole 2002. Movement of the pin 1402 can transition the release mechanism from the unreleased state to the released state.

Referring to FIG. 23, a perspective view of a docking cap of a biostimulator transport system having a release mechanism is shown in accordance with an embodiment. The crossbar release mechanism can include the docking cap 320 having several holes. A tether hole 2004 can be located in a proximal wall of the docking. Similarly, a tether hole 2004 can be located in a hub of the docking cap 320, adjacent to the hole in the proximal wall. The tether holes 2004 can be offset from each other along the wall. The fiber tether 1004 can extend distally through one or more of the tether holes 2004. The loop 1006 of the fiber tether 1004 can engage the release pin 1702 within the docking cavity of the docking, which is hidden by the docking cap 320 wall in FIG. 23. The release pin 1702 may therefore retain and/or release the loop 1006 to couple or decouple the biostimulator 100 from the biostimulator transport system 300.

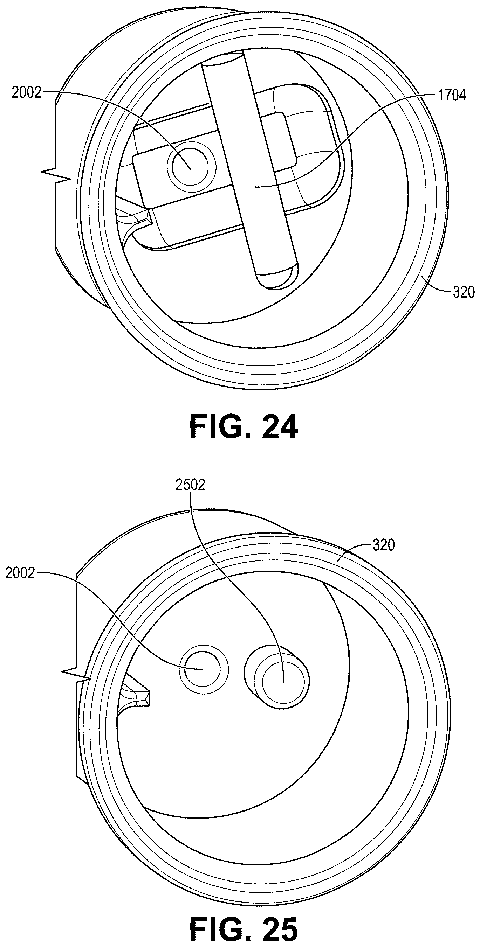

Referring to FIG. 24, a perspective view of a docking cap of a biostimulator transport system having a release mechanism is shown in accordance with an embodiment. The docking cap 320 can include the crossbar 1704 welded into a slot in the docking cavity. More particularly, the docking cap 320 can be formed as a multi-component structure having a crossmember or crossbar 1704 fit into a slot that extends laterally through the cavity. When received in the slot, the crossbar 1704 can extend across the cavity. The crossbar 1704 can be fixed in the slot by a weld or alternative material joint. The pin hole 2002 can extend axially through the docking cap 320, and can be laterally offset from the crossbar 1704. In operation, the fiber tether 1004 can loop around the crossbar 1704, to engage or disengage from the pin 1402 extending through the pin hole 2002. Movement of the pin 1402 can transition the release mechanism from the unreleased state to the released state.

Referring to FIG. 25, a perspective view of a docking cap of a biostimulator transport system having a release mechanism is shown in accordance with an embodiment. As described below, the release mechanism can include the fiber tether 1004 looping around a feature in the docking cap 320 and then connecting to a mandrel internal to the catheter shaft of the mandrel. The docking cap 320 can include a post 2502 extending distally from a proximal face of the docking cap 320 within the docking cavity. The post 2502 can be laterally offset from the pin hole 2002.

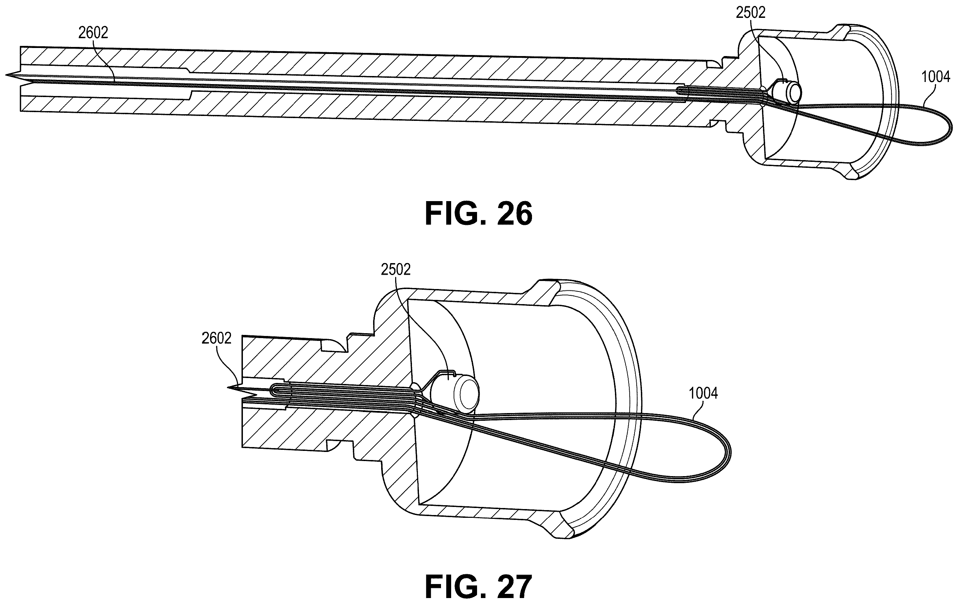

Referring to FIG. 26, a perspective view of a biostimulator transport system in an unreleased state is shown in accordance with an embodiment. The fiber tether 1004 can loop around the post 2502 within the docking cavity, and can extend proximally through the catheter shaft to connect to a mandrel 2602. The mandrel 2602 can be actuated by a handle mechanism to create tension within the fiber tether 1004. The tension can pull the fiber tether 1004 tight around the post 2502. When tightened, the fiber tether 1004 can remain in place on the stationary post 2502.

Referring to FIG. 27, a perspective view of a biostimulator transport system in an unreleased state is shown in accordance with an embodiment. The mandrel 2602 can include a hypotube having a short suture crimped to it. The tube can be connected to the release mechanism in the handle 304. A long loop can be fed through this loop and pulled into the shaft during docking. The loop can extend around and connect to the attachment feature 220. To release the attachment feature 220, the tube can be moved forward, e.g., distally, to allow the long suture to relax tension in the fiber tether 1004 and to allow the fiber tether 1004 to slide off of the post 2502. When released from the post 2502, the loop can extend distally to provide more room to fall away from the biostimulator 100. The biostimulator 100 may therefore be released from the biostimulator transport system 300.

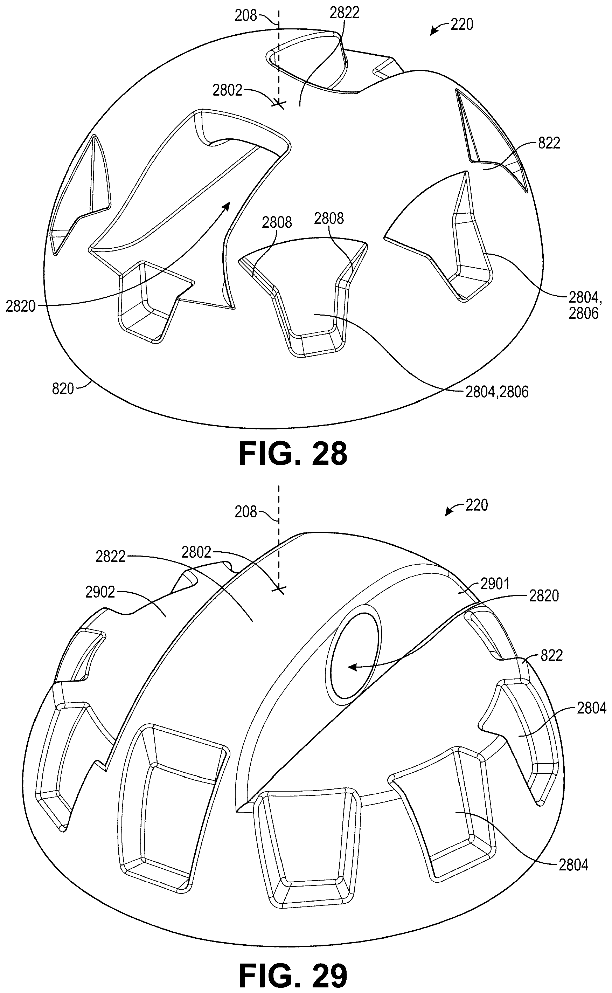

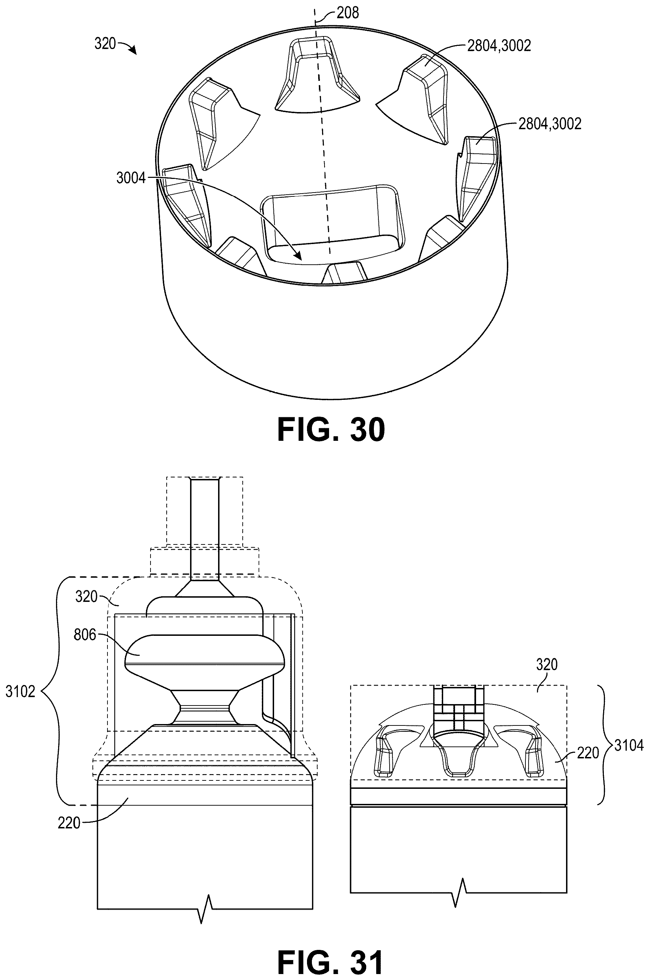

Referring to FIG. 28, a perspective view of an attachment feature of a biostimulator is shown in accordance with an embodiment. The attachment feature 220 can have a low profile height, for example, as compared to the attachment feature design having the button 806 (FIGS. 8-9). A height comparison is described in more detail with respect to FIG. 31, however, it will be appreciated that the low profile attachment feature 220 can have a generally domed shape between a distal edge or lip 820 that mounts on the housing and an apex 2802. The apex 2802 can be a proximalmost location on the attachment feature 220. The domed shape can include a hemispherical profile that includes one or more slots, grooves, or holes to facilitate various functions, as described below.

In an embodiment, the outer shoulder 822 is located around the longitudinal axis at an axial location between the distal edge 820 and the apex 2802. Several clutch elements 2804 can be distributed along the outer shoulder 822. For example, the clutch elements 2804 can include slots 2806 to receive corresponding clutch elements of the biostimulator transport system 300. The clutch elements 2804 of the biostimulator 100 can engage the corresponding clutch elements of the biostimulator transport system 300 to allow torque to be transferred to the helix distal end to drive into the target tissue.

In an embodiment, the clutch elements 2804 are evenly distributed about the longitudinal axis 208. For example, the attachment feature 220 can include two or more slots 2806 located along a circumference of the outer shoulder 822, and radial lines extending radially from the longitudinal axis 208 through the slots 2806 can be rotationally separated from each other about the longitudinal axis by a same angle. In an embodiment having two slots 2806, the slots can be separated by 180° about the longitudinal axis 208. In an embodiment having three slots 2806, the slots can be separated by 120° about the longitudinal axis 208, and so on.

The slots 2806 in the outer shoulder 822 can be sized and shaped to receive the corresponding clutch elements 2804 of the biostimulator transport system 300 and, thus, the size and shape may vary. The slot 2806 can be defined by a recessed depth below the outermost surface of the attachment feature 220 For example, the recess of the slots 2806 can define a slot edge 2808 extending around the slot 2806. The slot edge 2808 can define the shape of the slot 2806. For example, the slot edge 2808 can have slanted portions forming a V-shaped region of the slots 2806, a substantially vertical portion forming a bottom of the slot 2806, etc.

The regions of the slot 2806 can provide functional characteristics. In an embodiment, the V-shaped region of the slot 2806, which is between the slot edges 2808 tapering such that a circumferential distance between the slot edges 2808 is greater at a proximal location than a distal location, can receive and guide corresponding clutch elements 2804 of the biostimulator transport system 300 into the bottom of the slot 2806. The wider groove at the proximal end of the slot 2806 can capture the corresponding clutch elements 2804, and the element can slide over the tapered slot edge 2808 to be moved axial in line with the bottom of the slot 2806. The bottom of the slot 2806 can conform closely to corresponding clutch elements 2804. Accordingly, when the clutch elements 2804 are guided into the bottom, rotation of the docking cap 320 can press against and transfer torque to the attachment feature 220 via the slot 2806.

A depth of the slot 2806 can vary or taper in a distal direction. For example, a depth of the slot 2806 between the V-shaped region can be less than a depth of the slot 2806 between the bottom. The sloped internal surface of the slot 2806 can guide the clutch elements 2804 of the docking cap 320 gradually into place within the bottom of the slot 2806. Accordingly, both the slot edge 2808 and the internal surface of the slot 2806 can act to guide the corresponding clutch elements 2804 to a position at which torque can be effectively transmitted from the docking cap 320 to the attachment feature 220.

The attachment feature 220 can include a feature to receive a tether of the biostimulator transport system 300. For example, the attachment feature 220 can include a channel 2820 extending laterally through the dome-shaped outer surface. A bridge 2822 can extend over the channel 2820, and the apex 2802 can be located on the bridge 2822. The channel 2820 can allow for the tether, e.g., a flexible thread or fiber, to be passed through the channel 2820 beneath the bridge 2822. The tether can retain the attachment feature 220, and the biostimulator 100, relative to the biostimulator transport system 300 when looped around the bridge 2822. A tension can be applied to the tether to pull the attachment feature 220 into a docking volume of the docking cap 320.

Referring to FIG. 29, a perspective view of an attachment feature of a biostimulator is shown in accordance with an embodiment. The attachment feature 220 can include a vertical wall 2901 extending upward from a proximal face 2902 of the slotted shoulder. More particularly, the clutch elements 2804 can be distributed along the outer shoulder 822 distal to the proximal face 2902 of the attachment feature 220. The vertical wall 2901 can extend upward from the proximal face 2902.

In an embodiment, a hole extends laterally through the vertical wall 2901 forming the channel 2820 below the bridge 2822. The hole provides a passage through which a tether may be threaded. More particularly, the tether can include a flexible fiber or thread that can extend through the hole and retain the attachment feature 220 and biostimulator 100. The attachment feature 220 can be pulled into the docking cap 320 by the tether to engage the clutch elements of the attachment feature to the corresponding clutch elements 2804 of the docking cap 320.

Referring to FIG. 30, a perspective view of a docking cap of a biostimulator transport system is shown in accordance with an embodiment. The torquing mechanism of the biostimulator system 400 can include the clutch elements of the attachment feature 220 and corresponding clutch elements 2804 of the docking cap 320. The clutch elements 2804 of the docking cap 320 can correspond in size and shape to the clutch elements of the attachment feature 220. For example, whereas the attachment feature 220 includes slots 2806 (FIGS. 28-29) having a particular size and shape, the docking cap 320 can include one or more dogs 3002 that have a negative form of the slots 2806. More particularly, the dogs 3002 of the docking cap 320 can be sized and shaped to fill the space of the slots 2806 in the attachment feature 220.

The dogs 3002 act as teeth to engage the slots 2806 and, thus, torque applied to the docking cap 320 can drive rotation of the attachment feature 220. More particularly, when the attachment feature 220 is pulled back via a handle mechanism that applies tension to the tether, the clutch elements 2804 of the attachment feature and docking cap 320 align with each other. The alignment may be facilitated by a chamfer shape of the elements. For example, the slot edge 2808 or the tapered surface of the slot 2806 can guide the dogs 3002 into place. A robust mate between the components is established, and torque can be efficiently transmitted through the components to drive the biostimulator 100 into the target tissue.

The clutch elements 2804 of the biostimulator 100 and biostimulator transport system 300 are complementary and may be exchanged. More particularly, rather than having several slots 2806 as the clutch elements 2804, the base 802 of the biostimulator 100 can include several dogs 3002 distributed about the longitudinal axis 208 and the docking cap 320 can include several slots 2806 to receive the dogs 3002. Accordingly, the configuration of the clutch elements 2804 is illustrated by way of example and not limitation.

In an embodiment, the docking cap 320 includes a tether opening 3004 along the longitudinal axis 208. The tether opening 3004 provides a passage for the tether to extend through. For example, the opening can be at a center of the docking cap 320 to allow the tether to extend distally and pass through the docking cap 320 to attach to the biostimulator 100. The tether can loop through the hole formed below the bridge 2822 or in the vertical wall 2901 of the attachment feature 220. The tethers can therefore connect the handle to the attachment feature 220 through the docking cap 320 to apply the tension that docks the biostimulator 100 in the docking cap and engages the mating clutch elements 2804. The docking cap 320 may be attached to a bearing housing that allows the docking cap to spin independently of the deflectable catheter of the biostimulator transport system 300.

Referring to FIG. 31, a height comparison of various attachment features of a biostimulator is shown in accordance with an embodiment. The height of the combined attachment feature 220 and docking cap 320 can be reduced through the incorporation of meshing features that allow for torque to be more efficiently transferred to the biostimulator 100. In the example shown at left, the docking cap 320 can engage an attachment feature 220 having a button 806. The configuration can be similar to that shown in FIGS. 8-9. The button 806 can require sufficient volume in the docking cap 320 to be captured such that torque can be transmitted to the button 806 via the docking cap 320. Accordingly, the configuration of the assembly having the button 806 can have a first height 3102. The first height 3102 may be 0.272 inch, for example.

In contrast to the configuration having the button 806, the assembly having no button 806 and instead including the attachment feature 220 and docking cap 320 designs shown in FIGS. 28-30 can have a second height 3104. The attachment feature 220 having the neck 804 and the button 806 can have a length of 0.202 inch and the attachment feature having the domed shape can have a length of 0.118 inch, resulting in a length reduction of 0.084 inch. As a result of the length reduction, the second height 3104 may be less than the first height 3102 because the docking volume of the docking cap 320 can be reduced. For example, the second height 3104 may be 0.141 inch. An overall height reduction of 0.131 inch may therefore be realized by incorporating clutch elements 2804 that efficiently transfer torque and allow for the elimination of the button 806 and/or neck 804.

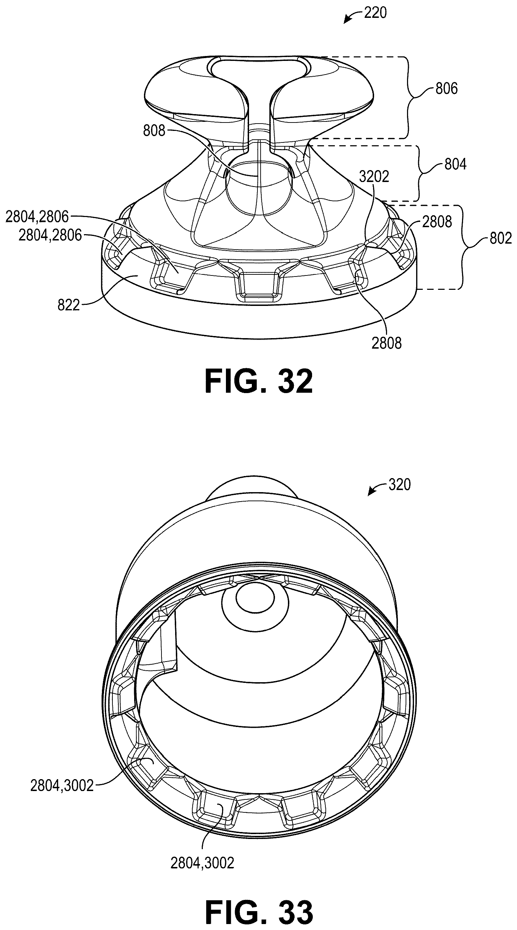

Referring to FIG. 32, a perspective view of an attachment feature of a biostimulator is shown in accordance with an embodiment. The attachment feature 220 can have a configuration similar to that shown in FIGS. 8-9. More particularly, the attachment feature 220 can include the base 802, the neck 804, and the button 806. Furthermore, the attachment feature 220 can include the lateral slot 808 and similar channels to receive the locking ball and tether. Although the inclusion of the button 806 can extend the overall height of the attachment feature 220, it may be beneficial for retrievability. More particularly, the button 806 can provide a feature to grasp for retrieval of the biostimulator 100 from the target anatomy. In addition, the button 806 allows for the lateral slot 808 to be formed, which facilitates delivery via the ball and tether mechanism. Accordingly, the attachment feature 220 can benefit from the inclusion of the neck 804 and/or button 806 despite the increased height.

In an embodiment, the attachment feature 220 includes the clutch elements 2804, e.g., slots 2806, located around the outer shoulder 822. The slots 2806 can be configured similar to those described with respect to the dome shaped attachment feature 220 above. More particularly, the slots 2806 can be evenly distributed about the longitudinal axis along the outer shoulder 822. In an embodiment, the slot edges 2808 of the cavities can touch at a proximal termination point 3202. The proximal termination point 3202 can be a proximalmost location of the slot edges 2808. The slot edges 2808 meet at the proximal termination point 3202. As a result, the slots 2806 can begin at the same point and taper away from each other in the distal direction. The tapering allows the corresponding dog 3002, when it engages one slot or the other, to be guided distally. As the dog 3002 is guided distally, the other dogs 3002 of the clutch elements 2804 can be rotated into place to mesh with the distributed slots 2806. The touching of the slots 2806 at the proximal termination point 3202 can ensure that the dog 3002 will engage one slot 2806, regardless of whether the slots 2806 are aligned with the dogs 3002 when the attachment feature 220 is pulled back into the docking cap 320.

Referring to FIG. 33, a perspective view of a docking cap of a biostimulator transport system is shown in accordance with an embodiment. The rotationally independent engagement of the slotted attachment feature 220 and the docking cap 320 can be facilitated by the docking cap having similarly sized and shaped dogs 3002. For example, the dogs 3002 of the docking cap 320 can have profiled edges that extend proximally to meet at another proximal termination. The profiled edges can match the profile of the slot edges 2808 and, thus, the dogs 3002 can fit and mesh with the slots 2806. When the attachment feature 220 is pulled into the docking cap 320, the dogs 3002 can therefore efficiently transmit torque to the attachment feature at the surfaces defining the slots 2806 to cause the biostimulator to rotate and engage the target tissue.

## Claims

1. A biostimulator transport system (300), comprising:
a locking tube (402) having a central lumen; and
a tether (602) extending through the central lumen, wherein the tether (602) includes a locking end (604) coupled to a tether body (606), and wherein the locking end (604) is wider than the tether body (606), **characterized in that** the locking tube (402) has a distal tube section (502) having a conical profile (803).

2. The biostimulator transport system of claim 1, wherein the locking end (604) includes a locking ball (608).

3. The biostimulator transport system of claim 1 or 2, wherein the tether body (606) includes a fiber (610).

4. The biostimulator transport system of any one of claims 1 to 3, wherein the locking tube (402) includes the distal tube section (502) and a proximal tube section (504), wherein the distal tube section (502) is stiffer than the proximal tube section (504).

5. The biostimulator transport system of any one of claims 1 to 4, further comprising a compression member to bias the locking tube (402) distally relative to the tether (602).

6. The biostimulator transport system of any one of claims 1 to 5, further comprising a handle (304), wherein the handle (304) is coupled to the locking tube (402) and the tether (602) to provide relative movement between the distal tube section (502) of the locking tube (402) and the locking end (604) of the tether (602).

7. The biostimulator transport system of any one of claims 1 to 6, further comprising a docking cap (320) having a docking cavity to receive an attachment feature (220) of a biostimulator (100), and a torque shaft coupled to the docking cap (320), wherein the locking tube (402) extends through a shaft lumen of the torque shaft.

8. A biostimulator (100), comprising:
a housing (206) containing circuitry (210);
a fixation element (106) coupled to a distal end (215) of the housing (206); and
an attachment feature (220) coupled to a proximal end (221) of the housing (206), wherein the attachment feature (220) has a base (802), a neck (804), and a button (806), wherein the attachment feature (220) includes a locking channel (612) extending longitudinally through the button (806) and the neck (804), **characterized in that**
the attachment feature (220) includes a lateral slot (808) extending laterally to the locking channel (612) through the base (802), the neck (804), and the button (806); and
the locking channel (612) tapers inward in a distal direction through the button (806).

9. The biostimulator of claim 8, wherein a base portion (810) of the lateral slot (808) extending through the base (802) is wider than a neck portion (812) of the lateral slot (808) extending through the neck (804).

10. The biostimulator of claim 8 or 9, wherein the locking channel (612) is wider within the button (806) than within the neck (804).

11. The biostimulator of any one of claims 8 to 10, wherein the base (802) includes an outer shoulder (822) extending about a longitudinal axis (208) of the attachment feature (220), and wherein a plurality of clutch elements (2804) are distributed along the outer shoulder (822).

12. The biostimulator of claim 11, wherein the plurality of clutch elements (2804) are evenly distributed about the longitudinal axis (208).

13. A biostimulator system (400), comprising:
the biostimulator transport system (300) of any one of claims 1 to 7; and
the biostimulator (100) of any one of claims 8 to 12 mounted on the biostimulator transport system (300).

## Patentansprüche

1. Biostimulatortransportsystem (300), umfassend:
ein Verriegelungsrohr (402), das ein zentrales Lumen aufweist; und
ein Halteseil (602), das sich durch das zentrale Lumen erstreckt, wobei das Halteseil (602) ein Verriegelungsende (604) beinhaltet, das an einen Halteseilkörper (606) gekoppelt ist, und wobei das Verriegelungsende (604) breiter als der Halteseilkörper (606) ist, **dadurch gekennzeichnet, dass** das Verriegelungsrohr (402) einen distalen Rohrabschnitt (502) mit einem konischen Profil (803) aufweist.

2. Biostimulatortransportsystem nach Anspruch 1, wobei das Verriegelungsende (604) eine Verriegelungskugel (608) beinhaltet.

3. Biostimulatortransportsystem nach Anspruch 1 oder 2, wobei der Halteseilkörper (606) eine Faser (610) beinhaltet.

4. Biostimulatortransportsystem nach einem der Ansprüche 1 bis 3, wobei das Verriegelungsrohr (402) den distalen Rohrabschnitt (502) und einen proximalen Rohrabschnitt (504) beinhaltet, wobei der distale Rohrabschnitt (502) steifer als der proximale Rohrabschnitt (504) ist.

5. Biostimulatortransportsystem nach einem der Ansprüche 1 bis 4, ferner umfassend ein Kompressionselement, um das Verriegelungsrohr (402) distal relativ zu dem Halteseil (602) vorzuspannen.

6. Biostimulatortransportsystem nach einem der Ansprüche 1 bis 5, ferner umfassend einen Griff (304), wobei der Griff (304) an das Verriegelungsrohr (402) und das Halteseil (602) gekoppelt ist, um eine relative Bewegung zwischen dem distalen Rohrabschnitt (502) des Verriegelungsrohrs (402) und dem Verriegelungsende (604) des Halteseils (602) bereitzustellen.

7. Biostimulatortransportsystem nach einem der Ansprüche 1 bis 6, ferner umfassend eine Andockkappe (320), die einen Andockhohlraum aufweist, um ein Befestigungsmerkmal (220) eines Biostimulators (100) aufzunehmen, und eine Drehmomentwelle, die an die Andockkappe (320) gekoppelt ist, wobei sich das Verriegelungsrohr (402) durch ein Wellenlumen der Drehmomentwelle erstreckt.

8. Biostimulator (100), umfassend:
ein Gehäuse (206), das eine Schaltung (210) enthält;
ein Fixierungselement (106), das an ein distales Ende (215) des Gehäuses (206) gekoppelt ist; und
ein Befestigungsmerkmal (220), das an ein proximales Ende (221) des Gehäuses (206) gekoppelt ist, wobei das Befestigungsmerkmal (220) eine Basis (802), einen Hals (804) und eine Taste (806) aufweist, wobei das Befestigungsmerkmal (220) einen Verriegelungskanal (612) beinhaltet, der sich in Längsrichtung durch die Taste (806) und den Hals (804) erstreckt, **dadurch gekennzeichnet, dass**
das Befestigungsmerkmal (220) einen seitlichen Schlitz (808) beinhaltet, der sich seitlich durch die Basis (802), den Hals (804) und die Taste (806) zu dem Verriegelungskanal (612) erstreckt; und
sich der Verriegelungskanal (612) nach innen in einer distalen Richtung durch die Taste (806) verjüngt.

9. Biostimulator nach Anspruch 8, wobei ein Basisabschnitt (810) des seitlichen Schlitzes (808), der sich durch die Basis (802) erstreckt, breiter als ein Halsabschnitt (812) des seitlichen Schlitzes (808), der sich durch den Hals (804) erstreckt, ist.

10. Biostimulator nach Anspruch 8 oder 9, wobei der Verriegelungskanal (612) innerhalb der Taste (806) breiter als innerhalb des Halses (804) ist.

11. Biostimulator nach einem der Ansprüche 8 bis 10, wobei die Basis (802) eine äußere Schulter (822) beinhaltet, die sich um eine Längsachse (208) des Befestigungsmerkmals (220) erstreckt, und wobei eine Vielzahl von Kupplungselementen (2804) entlang der äußeren Schulter (822) verteilt ist.

12. Biostimulator nach Anspruch 11, wobei die Vielzahl von Kupplungselementen (2804) gleichmäßig um die Längsachse (208) verteilt ist.

13. Biostimulatorsystem (400), umfassend:
das Biostimulatortransportsystem (300) nach einem der Ansprüche 1 bis 7; und
den Biostimulator (100) nach einem der Ansprüche 8 bis 12, der an dem Biostimulatortransportsystem (300) montiert ist.

## Revendications

1. Système de transport de biostimulateur (300), comprenant :
un tube de verrouillage (402) présentant une lumière centrale ; et
une attache (602) s'étendant à travers la lumière centrale, dans lequel l'attache (602) comprend une extrémité de verrouillage (604) couplée à un corps d'attache (606), et dans lequel l'extrémité de verrouillage (604) est plus large que le corps d'attache (606), **caractérisé en ce que** le tube de verrouillage (402) présente une section de tube distale (502) ayant un profil conique (803).

2. Système de transport de biostimulateur selon la revendication 1, dans lequel l'extrémité de verrouillage (604) comprend une bille de verrouillage (608).

3. Système de transport de biostimulateur selon la revendication 1 ou 2, dans lequel le corps d'attache (606) comprend une fibre (610).

4. Système de transport de biostimulateur selon l'une quelconque des revendications 1 à 3, dans lequel le tube de verrouillage (402) comprend la section de tube distale (502) et une section de tube proximale (504), dans lequel la section de tube distale (502) est plus rigide que la section de tube proximale (504).

5. Système de transport de biostimulateur selon l'une quelconque des revendications 1 à 4, comprenant en outre un élément de compression pour solliciter le tube de verrouillage (402) dans une direction distale par rapport à l'attache (602).

6. Système de transport de biostimulateur selon l'une quelconque des revendications 1 à 5, comprenant en outre une poignée (304), dans lequel la poignée (304) est couplée au tube de verrouillage (402) et à l'attache (602) pour induire un mouvement relatif entre la section de tube distale (502) du tube de verrouillage (402) et l'extrémité de verrouillage (604) de l'attache (602).

7. Système de transport de biostimulateur selon l'une quelconque des revendications 1 à 6, comprenant en outre un capuchon d'accueil (320) présentant une cavité d'accueil pour recevoir un élément d'attache (220) d'un biostimulateur (100), et un arbre de transmission de couple couplé au capuchon d'accueil (320), dans lequel le tube de verrouillage (402) s'étend à travers une lumière d'arbre de l'arbre de transmission de couple.

8. Biostimulateur (100), comprenant :
un boîtier (206) contenant un ensemble de circuits (210) ;
un élément de fixation (106) couplé à une extrémité distale (215) du boîtier (206) ; et
un élément d'attache (220) couplé à une extrémité proximale (221) du boîtier (206), dans lequel l'élément d'attache (220) présente une base (802), un col (804) et un bouton (806), dans lequel l'élément d'attache (220) comprend un canal de verrouillage (612) s'étendant longitudinalement à travers le bouton (806) et le col (804), **caractérisé en ce que**
l'élément d'attache (220) comprend une fente latérale (808) s'étendant latéralement par rapport au canal de verrouillage (612) à travers la base (802), le col (804) et le bouton (806) ; et
le canal de verrouillage (612) s'effile vers l'intérieur dans une direction distale à travers le bouton (806).

9. Biostimulateur selon la revendication 8, dans lequel une partie de base (810) de la fente latérale (808) s'étendant à travers la base (802) est plus large qu'une partie de col (812) de la fente latérale (808) s'étendant à travers le col (804).

10. Biostimulateur selon la revendication 8 ou 9, dans lequel le canal de verrouillage (612) est plus large à l'intérieur du bouton (806) qu'à l'intérieur du col (804).

11. Biostimulateur selon l'une quelconque des revendications 8 à 10, dans lequel la base (802) comprend un épaulement externe (822) s'étendant autour d'un axe longitudinal (208) de l'élément d'attache (220), et dans lequel une pluralité d'éléments d'embrayage (2804) sont répartis le long de l'épaulement externe (822).

12. Biostimulateur selon la revendication 11, dans lequel la pluralité d'éléments d'embrayage (2804) sont répartis uniformément autour de l'axe longitudinal (208).

13. Système de biostimulateur (400), comprenant :
le système de transport de biostimulateur (300) selon l'une quelconque des revendications 1 à 7 ; et
le biostimulateur (100) selon l'une quelconque des revendications 8 à 12 monté sur le système de transport de biostimulateur (300).
